Europäisches Patentamt

European Patent Office

Office européen des brevets

(11) Publication number: **0 153 292**
**B1**

(12) **EUROPEAN PATENT SPECIFICATION**

(45) Date of publication of patent specification: **22.03.89**   (51) Int. Cl.⁴: **C 07 C 51/47, C 07 C 57/10**

(21) Application number: **85870027.1**

(22) Date of filing: **15.02.85**

(54) **Purification of crude sorbic acid.**

(30) Priority: **16.02.84 US 580994**

(43) Date of publication of application:
**28.08.85 Bulletin 85/35**

(45) Publication of the grant of the patent:
**22.03.89 Bulletin 89/12**

(84) Designated Contracting States:
**BE DE GB**

(56) References cited:
**DE-A-1 618 357**
**DE-B-2 103 051**

(73) Proprietor: **MONSANTO COMPANY**
**Patent Department 800 North Lindbergh Boulevard**
**St. Louis, Missouri 63166 (US)**

(72) Inventor: **Brown, Henry Clay**
**1949 Spruce Street**
**St. Charles, MO 63303 (US)**
Inventor: **Crowley, Richard Paul**
**342 West Madison Avenue**
**Kirkwood, MO 63122 (US)**
Inventor: **Heintz, Daniel Nicholas**
**1436 Summerhaven Drive**
**St. Louis, MO 63146 (US)**
Inventor: **Ryland, James Robert III**
**23 Millbrook Lane**
**Kirkwood, MO 63122 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

EP 0 153 292 B1

Courier Press, Leamington Spa, England.

# EP 0 153 292 B1

**Description**

Background of the invention

This invention relates to the manufacture of sorbic acid and sorbic acid derivatives and more particularly to an improved process for producing sorbic acid and sorbic acid salts of high purity.

As disclosed, for example, in Fernholz et al. U.S. patent 3,022,342, sorbic acid may be produced by splitting a polyester that has been prepared by reaction of ketene and crotonaldehyde in an organic solvent in the presence of a zinc salt of a fatty acid. A variety of organic solvents may be used as the reaction medium in the formation of the polyester. Splitting of the polyester is accomplished by heating in the presence of an alkali. This causes the polymer to depolymerize and the free sorbic acid to be distilled out of the depolymerization reaction mass. Alternatively, as disclosed in Kunstle et al. U.S. patent 3,696,147, the polyester formed upon condensation of ketene and crotonaldehyde may be split in a reaction catalyzed by a mineral acid such as hydrochloric acid, sorbic acid precipitating from the hydrolysis reaction mixture.

In either case, the crude sorbic acid product is contaminated with impurities or "tars" formed in the condensation and/or depolymerization reaction. Certain of these tars are water-soluble while others are insoluble. As disclosed in the Kunstle et al. patent, the crude sorbic acid product may be purified by recrystallization from water. Water-soluble impurities are concentrated in the aqueous phase and insoluble impurities may be removed by centrifugation after the sorbic acid product is dissolved in water. Impurities may also be removed in part by carbon treatment of a crude sorbic acid in solution.

Where a sorbic acid is purified by carbon treatment, a relatively substantial load is placed on the carbon treatment facilities. When purification involves recrystallization from water, a substantial volume of water must be processed and energy consumption per unit of sorbic acid product is relatively high.

Commercially, there is a substantial demand for both sorbic acid and alkali metal sorbates such as potassium sorbate. In accordance with conventional practice, alkali metal sorbates are produced by caustic neutralization of the product obtained by subjecting crude sorbic acid to redissolution in water, carbon treatment and recrystallization. Thus, the energy requirements and the load placed on the carbon treatment facilities are high for the preparation of alkali metal sorbates as well.

Fernholz et al., patent 3,021,365 and Fernholz patent 3,499,029 both describe preparation of crude sorbic acid by thermal depolymerization, the process of the '029 patent accomplishing this by destructive distillation. The '365 patent describes conducting the thermal depolymerization in the presence of a high boiling diluent and a catalytic amount of an alkaline material. Acid catalyzed depolymerization of the polyester is disclosed as an alternative technique for producing crude sorbic. Also disclosed in the '365 patent is co-distillation of the depolymerization product in the presence of a solvent such as glycol or a monoalkylglycol ether. In the case of thermal depolymerization, the reference further discloses the use of volatile solvents such as carbon tetrachloride, petroleum ether or cyclohexane, either as an aid in removing the high boiling diluent from the crude sorbic acid product or in washing distilled sorbic acid.

Among the several objects of the present invention may be noted the provision of an improved process for producing a purified sorbic acid; the provision of such a process in which the crude sorbic acid product is produced by an acid catalyzed depolymerization of a polyester that is obtained by condensation of ketene and crotonaldehyde; the provision of such a process in which a portion of the free sorbic acid may be converted to an alkali or alkaline earth metal sorbate; the provision of such a process whose energy requirements for the production of alkali metal or alkaline earth sorbates are relatively low; and the provision of such a process in which the energy requirements for recovery of free sorbic acid may also be relatively low.

The present invention is directed to processes for the preparation of sorbic acid in which crotonaldehyde is reacted with ketene in the presence of a catalyst for the preparation of a polyester of 3-hydroxy-4-hexenoic acid and the polyester is depolymerized with an aqueous mineral acid to produce a crude crystalline sorbic acid precipitate.

In one process, the sorbic acid precipitate is separated from a supernatant aqueous phase. This process is characterised by contacting the crude crystalline sorbic acid with an organic solvent that comprises (i) a water-immiscible solvent selected from low molecular weight dihaloalkanes, low molecular weight trihaloalkanes, lower alkyl esters of low molecular weight alkanoic acids, low molecular weight ketones, and low molecular weight ethers, or (ii) a water-miscible solvent selected from lower molecular weight alcohols, lower molecular weight carboxylic acids, γ-butyrolactone, acetone and acetonitrile, said organic solvent being used in an amount which is effective for dissolving impurities formed in the preparation of the crude sorbic acid but under conditions such that no more than a minor proportion of said sorbic acid is dissolved, thereby producing a substantially purified crystalline sorbic acid and an organic liquor comprising said solvent and containing a substantial portion of said impurities; and mechanically separating said liquor from said crystalline sorbic acid.

A modified process of the invention is characterised by mixing an organic solvent that comprises a water-immiscible solvent selected from low molecular weight dihaloalkanes, low molecular weight trihaloalkanes, lower alkyl esters of low molecular weight alkanoic acids, low molecular weight ketones, and low molecular weight ethers, with an aqueous phase comprising the acidified aqueous phase of a depolymerization reaction mixture and containing crude crystalline sorbic acid, to contact said crude sorbic acid with said solvent which is effective for dissolving impurities formed in the preparation of the crude

2

sorbic acid and which will dissolve no more than a minor proportion of said sorbic acid thereby producing a slurry containing sorbic acid, said aqueous phase, and a mother liquor, said mother liquor comprising said solvent and containing impurities removed from said crude sorbic acid, and separating a substantially purified crystalline sorbic acid.

Brief description of the drawings

Fig. 1 of the drawings is a schematic flow sheet illustrating a crude sorbic acid purification system which may be used in implementing the improved process of the invention;

Fig. 2 is a schematic flowsheet illustrating an alternative version of the process of Fig. 1; and

Fig. 3 is a schematic flowsheet illustrating a further alternative embodiment of the process of the invention.

Description of the preferred embodiments

In accordance with the process of the invention, crude sorbic acid is preferably produced by splitting, i.e., depolymerization, of the polyester obtained from condensation of ketene and crotonaldehyde, and the resultant sorbic acid precipitate is contacted with an organic solvent for removal of impurities. Preferably, splitting of the polyester is carried out using an aqueous mineral acid as the depolymerization reagent. It has been discovered that contacting the solid phase crude sorbic acid precipitate with the solvent is effective for removing a very high proportion of the impurities, thereby substantially reducing the burden on the downstream steps (e.g., carbon treatment and recrystallization) used in producing a purified sorbic acid product. The impurities, and most particularly the water-insoluble impurities, have been found to concentrate on the surface of the crystalline precipitate obtained in the acid catalyzed splitting of poly(3-hydroxy-4-hexanoate), so that they are accessible for removal by contacting the precipitate with the organic solvent. Such topical application of solvent to the solid crude crystalline product has been found to remove a very substantial proportion of the impurities from the crude. As discussed hereinbelow, this technique not only facilitates the production of a high purity product with minimal burden on subsequent purification procedures, but also provides opportunities for substantial reductions in energy requirements, particularly where a significant portion of the sorbic acid is converted to an alkali or alkaline earth metal sorbate.

In each of the various embodiments of the process of the invention, contacting the crude crystalline sorbic acid with the organic solvent produces a substantially purified crystalline sorbic acid and an organic liquor containing impurities removed from the crude sorbic acid. In certain preferred embodiments, the crude sorbic acid is mixed with a slurry medium comprised of the organic solvent to produce a slurry comprised of the crystalline sorbic acid and a mother liquor, the mother liquor comprising the solvent and a substantial portion of the impurities removed from the crude sorbic. Sorbic acid crystals are separated from the mother liquor, preferably by mechanical means such as filtration, centrifugation or sedimentation, and the separated crystals preferably washed with further amounts of solvent. Alternatively, the crude sorbic acid may be slurried in water and separated from the aqueous slurry to provide a cake which is washed with the solvent. Contact of the crude sorbic with an organic wash solvent produces a wash liquor that is then separated from the cake.

In the preparation of the crude sorbic acid, crotonaldehyde is reacted with ketene in the presence of a catalyst such as a zinc carboxylate, for example, a zinc fatty acid salt to produce the poly(3-hydroxy-4-hexenoate) intermediate polyester. Various catalysts useful in preparing such polymers of crotonaldehyde and ketene are disclosed in M. A. Ikrina and V. D. Simonov, "Sorbic Acid and its Derivatives", published by KHIMIYA, Moscow, Nov. 1970, the pertinent portions of which are incorporated herein by reference. The polyester is then treated with an aqueous mineral acid, for example, concentrated hydrochloric acid, at moderately elevated temperature to produce a sorbic acid precipitate that is slurried in the aqueous reaction medium. The crude sorbic acid may be separated from this aqueous slurry by filtration or centrifugation.

Examples of water-miscible solvents which may be used in the process of the invention are acetone, acetonitrile, various lower alcohols such as methanol, ethanol, or isopropanol, and solutions of acetone, acetonitrile, or lower alcohols in water. Preferably, however, a water-immiscible solvent is employed. Among these are dihaloalkanes such as dichloromethane, dibromomethane, 1,1 - dichloroethane, 1,2 - dichloroethane, 1,1 - dibromoethane, or 1,1 - dichloropropane, trihaloalkanes such as chloroform, 1,1,1 - trichloroethane or 1,1,2 - trichloroethane, lower alkyl ($C_1$ to $C_8$) esters of lower alkanoic acids ($C_1$ to $C_8$) such as ethyl acetate, ethyl propionate, or butyl formate, lower molecular weight ketones ($C_1$ to $C_{10}$) such as methyl ethyl ketone, methyl n-propyl ketone, methyl isobutyl ketone, or methyl isopropyl ketone, aromatic hydrocarbons such as toluene and xylene, and halogenated aromatic compounds such as chlorobenzene.

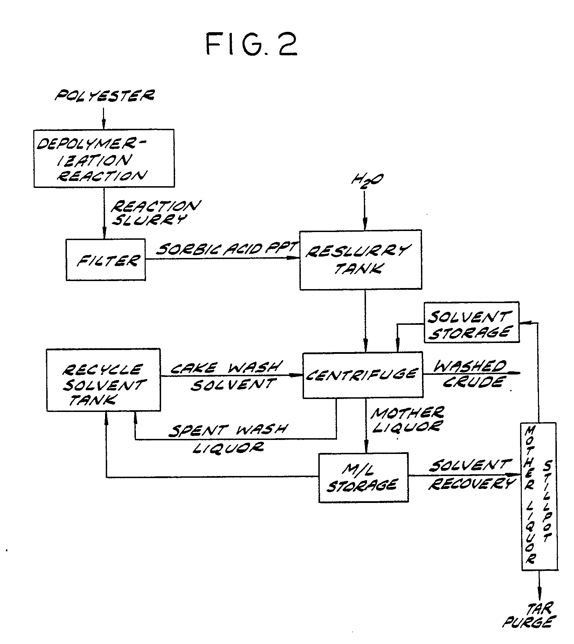

While the solvent used should be effective for dissolving impurities, yield and/or productivity may suffer if the solubility of sorbic acid is appreciably greater than the solubility of the impurities. Dihaloalkane and trihaloalkane solvents are particularly preferred because of the relatively high solubility of impurities and low solubility of sorbic acid therein. Although the solubility or sorbic acid in water-miscible solvents is generally greater than in water-immiscible solvents, the former are suitable for applications such as that illustrated in Fig. 2 where sorbic acid/solvent contact time is limited. But even in that case, no more than a minor portion of the sorbic acid should be dissolved in the solvent under the solvent treatment conditions.

For purposes of solvent recovery, and facilitating the purging of impurities, it is further preferred that the solvent either have a volatility greater than that of water or from an azeotrope having a greater volatility than water and containing a substantial mole fraction of solvent. Dichloromethane, which has a greater volatility than water and a specific gravity which differs substantially from that of water, is particularly preferred. Other particularly suitable solvents include ethyl acetate, methyl isobutyl ketone and 1,1,1-trichloroethane.

It is particularly preferred that the crude sorbic acid be subjected to a two-stage essentially countercurrent solvent treatment operation in which fresh solvent is used in the second stage, while recycle solvent contaminated with some level of impurities is used in the first stage. Hereinbelow, the organic liquor obtained in the first stage is referred to as a mother liquor, while the organic liquor obtained from the second stage is referred to as a spent wash liquor.

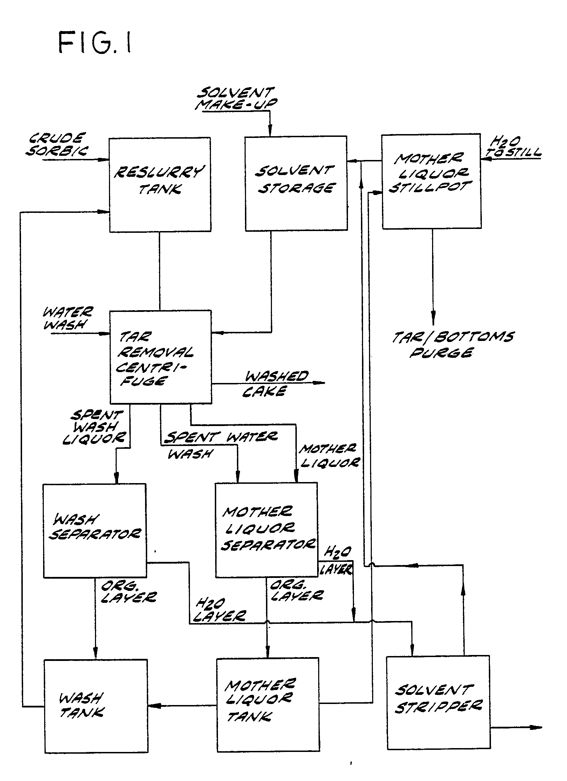

Fig. 1 illustrates a preferred flow sheet for implementation of the improved process of the invention using a water-immiscible solvent having a higher volatility than water (or which forms an azeotrope having a higher volatility than water). Crude sorbic is separated from the polyester depolymerization reaction slurry and mixed with a solvent in a reslurry tank. The slurry, which comprises crystalline sorbic acid and a mother liquor comprising the solvent and containing a substantial fraction of the impurities removed from the crude acid, is then delivered to a tar removal centrifuge. Mother liquor discharged from the centrifuge is directed to a mother liquor separator for separation of any residual aqueous phase. Prior to discharge of solids from the centrifuge, the centrifuge cake is washed, first with solvent and then with water. The secondary spent wash liquor obtained from the solvent wash is directed to a wash separator, while the spent water wash can be directed to either separator. The aqueous layer from the mother liquor separator is passed over to the wash separator and the organic phase delivered to a mother liquor hold tank. The organic layer from the wash liquor separator is recycled via a wash tank to the reslurry tank, while the aqueous phase is transferred to a stripper for recovery of residual solvent. Mother liquor from the mother liquor hold tank is divided. The major fraction, typically about two-thirds, is transferred to the wash tank and combined with the secondary spent wash liquor for recycle to the reslurry tank, while the remaining fraction of mother liquor is sent to a distillation pot. Distillation of mother liquor leaves a residue which can be purged from the process. Solvent stripped from the aqueous phase in the solvent recovery stripper and that separated from the impurities in the mother liquor still are returned to fresh solvent storage for use in the solvent wash of the centrifugation cake. Solvent is added to the storage tank to make up for losses.

In the reslurry step the solvent is preferably mixed with the crude sorbic acid at a temperature which is generally not critical but is preferably governed by solvency and volatility characteristics of the particular solvent used. Thus, the temperature should be maintained below that at which significant vapor losses are incurred and within a range where solubility of tars is relatively high while solubility of sorbic acid is not excessive. For halogenated solvents, ketones, esters and the like, the slurry temperature is preferably not greater than 30°C, more preferably not greater than 25°C. For other solvents, most particularly aromatics, a somewhat higher temperature is preferred in order to maximize solubility of tarry impurities. Conveniently, crude sorbic acid precipitate and solvent are mixed in such proportions as to provide a slurry having a solids content up to about 50%, preferably between about 20% by weight and about 40% by weight. Higher or lower concentrations may be used, with the optimum being a function of the absolute and relative solubilities of the impurities and sorbic acid. The relative amount of solvent should be sufficient to dissolve a high proportion of the impurities but not so great as to readily dissolve more than a minor portion of the sorbic acid crystals. Under steady-state conditions, where a significant portion of mother liquor is recycled, the sorbic acid content of the slurry medium tends to approach saturation, thereby inhibiting dissolution of any significant portion of the sorbic acid.

Although the crystalline sorbic acid may be separated from the slurry by filtration or any other solid/liquid separation technique, this step is preferably carried out by centrifugation. A peeler-type centrifuge is particularly advantageous for this purpose but other centrifuges, including variable speed basket centrifuges, continuous screen bowl, pusher, and solid bowl machines, can also be used. Preferably, a peeler-type centrifuge is rotated at an angular velocity sufficient to provide a radial acceleration of at least about 100 times the acceleration of gravity at the periphery of the basket or bowl. However, in the case of any basket centrifuge, the acceleration should be low enough to ensure that a uniform cake is formed over the basket surface.

After removal of the mother liquor containing the impurities, the cake is washed, first with fresh solvent and then with water. Conveniently, the cake may be sprayed with the wash liquids. In both cases the wash liquid should be delivered at such rate as to assure an effective displacement wash. The water wash is important in order to displace as much residual solvent as possible from the cake. Volumes of solvent and water wash may vary substantially with the type of centrifuge employed and the flow characteristics of the liquids through the cake. However, for a given system, the optimum volumes may be readily arrived at by those skilled in the art.

Considerations governing the preferred slurry temperature also apply to the solvent wash temperature. Although yield losses due to partial dissolution of crude sorbic may be controlled by recycle of the spent solvent wash liquor, dissolution of sorbic acid reduces the productivity of the centrifugation step.

As noted above, the mother liquor from the centrifuge is divided at the mother liquor hold tank, with

EP 0 153 292 B1

the larger fraction being recycled and the smaller fraction diverted to provide a purge of impurities from the system. Where the solvent used is more volatile than water, or forms an azeotrope more volatile than water, fresh solvent is readily recovered, and an aqueous purge stream provided, by distilling the mother liquor in the presence of water. Fresh solvent is distilled over and a bottom fraction is provided comprising water and the impurities.

Where the solvent used is water-immiscible but of low volatility (and forms no water azeotrope having a higher volatility than water), fresh solvent may be recovered from the mother liquor by distillation. Although an aqueous bottom fraction can be produced even from a high boiling mother liquor by use of steam distillation, energy consumption may be very high if volatility of the solvent is significantly lower than that of water.

Where the solvent is less volatile than water, it may not be feasible to recover residual solvent from the aqueous effluent, though at least partial recovery may be feasible where the solvent and water form a low boiling azeotrope.

While a water-immiscible solvent is preferably used when wet crude sorbic is processed according to the flowsheet of Fig. 1, a water-miscible solvent can be used advantageously in a comparable processing scheme if the crude sorbic is first dried or substantially dewatered.

Alternatively, solubility losses of sorbic acid in a water-miscible solvent may be minimized by applying the solvent directly to the centrifuge or filter cake obtained in the separation of the precipitate from the polyester depolymerization reaction mixture. However, in order to achieve maximum elimination of ionic chloride contamination where splitting of the polyester is carried out with hydrochloric acid, treatment of the crude sorbic with a water-miscible solvent is preferably carried out in accordance with the flow sheet illustrated in Fig. 2. As shown in this flow sheet, the sorbic acid precipitate is first separated from the aqueous phase of the depolymerization reaction mixture, for example, by filtration, the precipitate reslurried in fresh water, and the crude sorbic acid separated from the fresh water slurry by centrifugation. The centrifuge cake is then washed with the water-miscible solvent. Slurry concentrations and other conditions for the process of Fig. 2 are generally comparable to those for the process of Fig. 1.

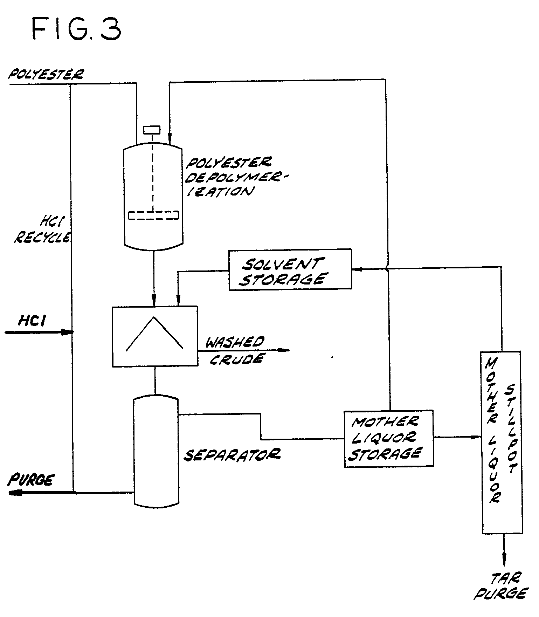

Illustrated in Fig. 3 is a further alternative embodiment of the invention which is implemented using a water-immiscible solvent. Prior to separation of the aqueous phase from the depolymerization reaction mixture, the crude sorbic acid is contacted with solvent by mixing the solvent with the reaction mixture in the depolymerization reactor. Sorbic acid is separated from the resulting slurry by centrifugation, or other liquid/solid separation means, the separated crystals washed with fresh solvent and water, and the two-phase filtrate directed to a separator from which part of the aqueous phase is recycled to the reactor and the organic liquor directed to a mother liquor hold tank. A major fraction of the mother liquor is recycled to the depolymerization reactor while the remainder is forwarded to a mother liquor still for recovery of solvent and purging of tarry impurities. Solvent recovered by distillation of the mother liquor is recycled to a solvent storage tank for use in washing the centrifuge cake. Preferably the amount of organic solvent and/or mother liquor used is sufficient to produce a sorbic acid slurry having a solids content of 20% to 40% by weight.

Regardless of what flowsheet is followed for solvent removal of impurities from the crystalline sorbic acid, the partially purified sorbic is preferably recrystallized from water and/or an organic solvent. After the solvent treated sorbic acid precipitate is dissolved in the recrystallization solvent, it is preferably treated with carbon for adsorption of residual impurities. Prior removal of most of the impurities by solvent treatment greatly reduces the impurity load on subsequent purification procedures.

When the sorbic acid is recrystallized from water, it may be desirable to conduct a further recrystallization, for example, from aqueous methanol.

Where the sorbic acid is to be converted to an alkali or alkaline earth metal sorbate, it has been found unnecessary to recrystallize the free acid after solvent treatment of the crude crystalline sorbic. Instead, the solvent-treated sorbic acid can be contacted with a basic alkali or alkaline earth metal compound in an aqueous medium to produce an aqueous solution of an alkali of alkaline earth metal sorbate. This solution may then be treated with carbon, after which the sorbate salt is recovered by conventional techniques. Thus, substantial energy savings are achieved by avoiding the recrystallization of the free sorbic acid in the process of preparing the salt.

In order to prepare an aqueous solution of sorbate salt, the solvent treated sorbic acid is slurried in water, and a basic aqueous solution of an alkaline earth or alkali metal compound, typically a hydroxide, is added to the slurry. The strength of the slurry and the basic solution are preferably controlled to provide sorbate salt solution strength of between about 30% and about 50% by weight after addition of base is complete. The amount of base added is preferably in substantial stoichiometric equivalence to the sorbic acid. The rate of neutralization should be controlled and/or the salt sorbate reaction mixture cooled to maintain its temperature below about 60°C, preferably below about 50°C, to prevent thermal degradation.

After the sorbate salt solution is prepared, it may be carbon-treated for further removal of impurities. As those skilled in the art will appreciate, various forms of activated carbon and contacting techniques can be utilized for carbon treatment, with the most appropriate system for a particular application being determinable by routine laboratory or plant experimentation. After carbon treatment, the sorbate salt may be recovered from the aqueous solution by conventional crystallization techniques.

The following examples illustrate the invention. In these examples the legend "pbw" indicates "parts

5

by weight". In all cases, the crude sorbic acid was produced by depolymerizing a ketene/crotonaldehyde polyester using 34—37% hydrochloric acid at 60—90°C. As a result of solvent treatment in these examples, the partially purified sorbic acid product had an assay of at least 99.3% sorbic acid, except where otherwise indicated.

Example 1

A portion (200 pbw) of the dry crude sorbic acid was wetted with water (40 pbw) and slurried in methyl isobutyl ketone (300 pbw). Sorbic acid crystals were separated from the resultant mother liquor by centrifugation and the centrifuge cake spray washed with water-saturated methyl isobutyl ketone (75 pbw) followed by water (75 pbw). This procedure was carried out four times with recycling of mother liquor in order to reach a steady state condition at which the mother liquor composition stabilized.

An additional portion of dry crude sorbic acid (200 pbw; 93.1% assay) was wetted with water (40 pbw) and slurried in a medium which comprised a combination of a portion of the mother liquor obtained at steady state (225 pbw) and a portion of the wash liquor (75 pbw) obtained by spray washing the immediately preceding batch of crude sorbic acid. This slurry was stirred for from five to six minutes, and the crystalline sorbic acid then separated from the mother liquor by centrifugation. The centrifuge cake was washed with a portion of fresh methyl isobutyl ketone (75 pbw) saturated with water), the spent wash liquor being maintained separate from the mother liquor. Thereafter, the centrifuge cake was washed with water (75 pbw). This procedure was carried out on four additional 200 pbw portions of crude sorbic acid, in each case recycling the methyl isobutyl ketone mother liquor and spent wash liquor from the preceding batch.

From operations after attainment of steady state, a total of 700 pbw (dry basis) washed sorbic acid was obtained, for an overall yield of 94.0%. Solids content of the mother liquor was found to be 24.5% by loss on drying, while the sorbic acid content of the mother liquor residue was found to be 38.1% by gas-liquid chromatographic analysis.

A portion of the mother liquor (173 pbw) was concentrated to 72.6 pbw by distilling off methyl isobutyl ketone at 90 mm to 100 mm Hg absolute pressure. The residue was cooled to 30°C and centrifuged. The centrifuge cake was washed with fresh methyl isobutyl ketone (15 pbw) and the resulting residue wash liquor obtained maintained separate from the concentrated mother liquor (i.e., the filtrate from the centrifugation). Both concentrated mother liquor and residue wash liquor were evaporated to dryness.

The dry centrifuge cake was found to weigh 9.9 pbw and contain 99% by weight sorbic acid; the residue from evaporating the concentrated mother liquor to dryness was found to weigh 22.8 pbw and to contain 15.5% sorbic acid; and the residue obtained by evaporating the spent wash liquor to dryness was found to weigh 7.4 pbw and to contain 33.2% sorbic acid. Thus, a total of 15.8 pbw sorbic acid was accounted for, which represented 98.3% of the sorbic acid content of the process mother liquor as determined by loss on drying.

Example 2

Using the method generally described in Example 1, crude crystalline sorbic acid was solvent treated by slurrying it in methyl isobutyl ketone and separating the crystalline product from the resulting mother liquor by centrifugation. The centrifuge cake was washed with fresh methyl isobutyl ketone, and spent wash liquor being maintained separate from the mother liquor. The mother liquor was recycled and used for slurrying additional portions of crude sorbic acid which were also separated by centrifugation. After several batches, a portion of mother liquor (500 pbw), in which the sum of the dissolved tar and sorbic acid contents had reached 20.2%, was subjected to azeotropic distillation for recovery of the methyl isobutyl ketone solvent. Distillation was conducted in a jacketed still pot provided with a mechanical stirrer, addition funnel, thermometer, ice water trap, and a temperature controlled hot oil supply to the jacket. A vapor line from the still pot was connected to a condenser and the condenser vented through an icetrap to a vacuum source.

In carrying out the distillation, tap water (300 pbw) was initially charged to the still pot and mother liquor (250 pbw) was charged to the addition funnel. The system was placed under a vacuum of 20 in. Hg and the oil bath heated to and maintained at a temperature of 115°C. When the water in the still pot had reached a temperature in the range of 60°C to 65°C, the discharge cock on the addition funnel was opened partially, and addition of mother liquor to the still pot commenced. Addition of mother liquor was carried out over a period of approximately one hour and 45 minutes. After all the mother liquor had been added, heating of the still pot was continued and the 20 in. Hg vacuum maintained. When the vapor temperature rose to about 57°C, it was observed that most of the distillate consisted of water and the distillation operation was, therefore, terminated.

Thereafter an additional portion of water (30 pbw) was charged to the still pot and another portion of mother liquor (250 pbw) distilled in the manner generally described above. In the second batch, however, addition of mother liquor was completed over a period of approximately 50 minutes. When the vapor temperature reached 55°C, pressure in the still pot was allowed to rise to approximately atmospheric and the heel was heated to 80°C. Thereafter, the pot was drained.

It was found that the total weight of distillate (both phases) from the first batch was 208.1 pbw and from the second batch 212.2 pbw. The total weight of the tar/sorbic acid/water heel collected from the pot after the second batch was 363.8 pbw. After drainage the still pot was washed and an additional 20.0 pbw of tar removed. Total recovery of materials was thus 807.3 pbw (including the 3.2 pbw in ice trap mentioned below), or 97.3% based on the total charge of 830 pbw.

The organic phase from the first batch weighed 176.3 pbw and from the second batch 178.2 pbw, while the aqueous layers weighed 31.8 pbw and 34 pbw, respectively. A total of 0.8 pbw of organic phase and 2.4 pbw or aqueous phase were recovered from the ice trap.

Example 3

Dry crude sorbic acid (200 pbw dry basis plus 40 pbw added water) was slurried in fresh ethyl acetate (300 pbw) and the sorbic acid crystals separated from the slurry by centrifugation. The centrifuge cake was washed first with water-saturated ethyl acetate (80 pbw) and then with water (80 pbw).

Mother liquor (300 pbw) obtained as the filtrate in the first batch centrifugation was used for slurrying an additional portion of sorbic acid (200 pbw dry basis plus 40 pbw water) and the resulting slurry centrifuged and the cake washed in the same manner as the first batch. Thereafter, ten additional batches (batches 3 to 12) of crude sorbic acid (200 pbw dry basis plus 40 pbw water) were subjected to solvent treatment by first slurrying them in a medium obtained by combining mother liquor (225 pbw) and spent cake wash liquor (75 pbw) from the previous batch, separating the crystalline sorbic from the slurry by centrifugation, and washing of the cake in the manner described above. Mother liquor in excess of 225 pbw from each batch was effectively purged from the system. To provide the desired volume of 300 pbw slurry medium, small portions of fresh ethyl acetate make-up solvent were added to batches 10 (5 pbw), 11 (12 pbw), and 12 (10 pbw).

A material balance in batches 7 through 12 showed a 94.7% recovery of sorbic acid.

Example 4

Crude sorbic acid was separated from the acid catalyzed depolymerization mixture by vacuum filtration. The filter cake was washed with methyl ethyl ketone, thus cleaning off tarry impurities and yielding a sandy-colored washed filter cake.

A portion of the washed sorbic acid (37.5 pbw) was added to a 40% solution of ethanol in water (150 g) and the resulting deep yellow solution heated to 70°C. An activated carbon (1.9 pbw) was mixed with the solution and the carbon slurry held for 15 minutes. Thereafter the carbon was filtered out of the solution yielding a pale yellow filtrate. Sorbic acid was recovered from the carbon-treated solution by crystallization. The final crystalline product was very white.

Another portion of the methyl ethyl ketone washed crude (25 pbw) was mixed with a 40% solution of ethanol in water (100 pbw) and the sorbic acid recrystallized without carbon treatment. The yellow crystals obtained in this case were substantially purified and of high assay.

Example 5

Two samples of crude sorbic acid crystalline filter cake (250 g each) were washed with a 95% solution of ethanol and water (100 ml). The filtrate (mother liquor) was preserved and designated "Filtrate A".

Another sample of dried crude sorbic acid (200 g) was placed on a Buchner funnel, slurried with water, and filtered sufficiently to provide a reasonably uniform cake. A portion of "Filtrate A" (100 ml) was sprayed onto the cake on the Buchner funnel, while suction was applied below the filter paper. The filtrate (mother liquor) obtained was retained and designated "Filtrate B". Thereafter the cake was washed with a portion of fresh 95% ethanol/water solution (100 ml). The filtrate (spent wash liquor) from the second stage wash was also retained and designated "Filtrate C". Analyses of Filtrates B and C are set forth in Table I.

TABLE I

|  | Sample | |
|---|---|---|
|  | Filtrate B | Filtrate C |
| Total Weight | 107.8 g | 139.6 g |
| Weight of Tars plus Sorbic Acid | 18.25 g | 5.52 g |
| Assay of Solids (% Sorbic Acid) | 51.3 | 36.0 |
| Composition of Filtrate: | | |
| Liquid (%) | 83.0 | 96.0 |
| Tar (%) | 8.3 | 2.5 |
| Sorbic Acid (%) | 8.9 | 1.4 |

Example 6

Crude sorbic acid was subjected to two-stage countercurrent solvent treatment using 95% ethanol as the solvent.

A portion of wet crude sorbic acid (200 g) was placed on a Buchner funnel. Water was added to reslurry the sorbic and distribute the cake over the filter paper. A portion of 95% ethanol/water solution (100 ml) was applied over the top of the cake in the form of a fine spray over a period of 30 seconds, while vacuum was applied to a receiving flask placed under the funnel. The cake on the funnel was thereafter washed for 30 seconds by spraying thereover a portion of fresh solvent (100 ml).

Another portion of wet crude sorbic acid (200 g) was delivered to a Buchner funnel. Sufficient water to slurry the crude was then added and filtered through the cake. After the water had passed through, the cake was washed with 95% ethanol solvent by spraying the solvent over the cake in the manner described above.

A further portion of wet crude sorbic acid (200 g) was slurried with water on a Buchner funnel as described above to provide a uniform cake, and then washed with a portion of the mother liquor that had been obtained in the two prior cake washing operations. Washing with mother liquor was carried out by spraying it over the cake. However, because of a somewhat retarded drain rate, the spray was interrupted for five seconds at ten-second intervals to provide an effective displacement wash.

A further portion of wet crude sorbic acid (250 g) was placed on the Buchner funnel and slurried with deionized water (about 800 ml). The water was removed under vacuum. The funnel containing the water-washed cake was then transferred to a second flask.

On the second flask, the cake was sprayed with recycled enthanol/tar (mother liquor) solution (100 ml) obtained from the prior cake washing operation. Washing was carried out on the following cycle: ten seconds spray; ten seconds rest; ten seconds spray; ten seconds rest; ten seconds spray; 30 seconds drainage. During the entire time, the receiving flask was maintained under vacuum to draw liquid through the cake.

After washing with the mother liquor, the cake was transferred to a third flask where it was sprayed with fresh 95% ethanol/water solution (100 ml) according to the same interrupted spray schedule used in the mother liquor cake wash.

Five additional 250 g samples of crude sorbic acid were slurried with water and filtered in a Buchner funnel on one flask, transferred in the funnel to a second flask, spray washed with recycled mother liquor, transferred to a third flask, and spray washed with fresh ethanol/water solution, all in the manner described above.

Mother liquor filtrates from the first stage wash (second funnel) were combined, analyzed, and found to contain approximately 24.4% by weight solids. A sample of the combined secondary spent wash liquor emanating from the second stage wash (third funnel) was analyzed and determined to contain 3.16% by weight solids.

Example 7

Wet crude sorbic acid precipitate (127 pbw; dry basis: 101.5 pbw and 93.5% by weight sorbic acid) was subjected to two stage solvent treatment with dichloromethane for removal of tars. The precipitate was first slurried with one portion of dicloromethane (230 pbw), the sorbic acid crystals were then recovered from the resulting mother liquor by filtration, and the filter cake washed with a further portion of dichloromethane (50 pbw).

This procedure was repeated using essentially the same quantities of wet crude but with 1,1,2-trichloroethane and 1,1,1-trichloroethane, respectively, as the solvent. In each instance, the washed filter cake was dried and weighed. Results of these runs are set forth in Table II.

TABLE II

| Identity of solvent | Quantity of solvent | | Weight of dry washed cake |
| --- | --- | --- | --- |
| | Slurry | Cake wash | |
| Dichloromethane | 230 pbw | 50 pbw | 83.2 pbw |
| 1,1,2-Trichloroethane | 230 pbw | 50 pbw | 86.1 pbw |
| 1,1,1-Trichloroethane | 230 pbw | 50 pbw | 86.7 pbw |

Wet crude sorbic acid prepared in the manner described above was slurried in water and then separated from the water phase by centrifugation. The centrifuge cake was spray washed with a solvent for removal of tars. Three runs were made, using ethyl alcohol, methyl isobutyl ketone, and 1,1,1-trichloroethane, respectively, as the washing solvent. In each case a 127 pbw sample of wet crude (dry basis: 101 pbw and 93,5% sorbic acid) was used. After washing, the centrifuge cake was weighed to determine the recovery of sorbic acid. Results of these runs are set forth in Table III.

## EP 0 153 292 B1

TABLE III

| Identity of solvent | Quantity of spray | Weight of dry washed cake |
|---|---|---|
| Ethyl Alcohol | 100 pbw | 80.0 pbw |
| Methyl Isobutyl Ketone | 100 pbw | 83.8 pbw |
| 1,1,1-Trichloroethane | 100 pbw | 92.2 pbw |

Substantial removal of tars was achieved with each of the solvents. While the color attained by use of ethyl alcohol was slightly better than that obtained with the other solvents, losses of sorbic acid by dissolution in the solvent were higher.

A further portion of dry crude sorbic acid (150 pbw, 93% sorbic acid) was slurried in water (850 pbw) containing NaCl (30 pbw). Sorbic acid crystals were separated from the slurry by centrifugation, and the centrifuge cake spray washed with ethyl alcohol (105 pbw). This procedure was repeated five times, all runs being conducted at room temperature, and the accumulated cake was dried and weighed. Total recovered sorbic acid was 568 pbw, a yield of 81.42%.

The above procedure was repeated five more times with washing being conducted at 10°C. An 86% recovery of sorbic acid was obtained.

Additional samples of dry crude sorbic acid (each 101.6 pbw containing 95 pbw sorbic acid) were spray or slurry treated with various solvents. The treated crude samples were thereafter dried and weighed. The results of these runs are set forth in Table IV.

TABLE IV

| Identity of solvent | Mode of treatment | Quantity of solvent | Sorbic acid recovery |
|---|---|---|---|
| Ethyl Alcohol | Spray | 100 pbw | 84.2% |
| 1,1,1-Trichloroethane | Slurry | 300 pbw | 91.3% |
| Dichloromethane | Slurry | 250 pbw | 87.6% |
| Methyl Isobutyl Ketone | Spray | 100 pbw | 88.2% |
| 1,1,1-Trichloroethane | Spray | 100 pbw | 97.4% |
| 1,1,2-Trichloroethane | Slurry | 300 pbw | 90.6% |
| 1,1,1-Trichloroethane | Spray | 200 pbw | 97.3% |

These results demonstrate that 1,1,1-trichloroethane and 1,1,2-trichloroethane are excellent solvents for removal of tars from sorbic acid by solvent treatment, and cause only relatively low sorbic acid losses. In all cases, slurry washed sorbic acid exhibited better color than spray washed cake.

Example 8

Various solvents were evaluated at room temperature for use as a slurry and wash medium for removing tarry impurities from crude sorbic acid. Each solvent was tested by slurrying wet crude sorbic acid (120 pbw) in solvent (200 pbw), separating the crystalline sorbic acid from the solvent by centrifugation, spray washing the centrifuge cake with solvent (50 pbw) and then water (50 pbw) and air-drying the washed cake. The solvents tested and the results are set forth below:

| Homologous series | Homologs | Water misc. | Tar solvency |
|---|---|---|---|
| Alcohols | Methanol | Yes | Good |
| | Ethanol | Yes | Good |
| | Isopropanol | Yes | Good |
| | n-Propanol | Yes | Good |
| Esters | Methyl acetate | Mod. | Good |
| | Ethyl acetate | No | Good |
| | γ-butyrolactone | Yes | Good |
| Ethers | Tetrahydrofuran | Yes | Good |
| | Isopropyl ether | No | Moderate |
| | 1,2-dimethoxyethane | No | Good |
| Ketones | Acetone | Yes | Good |
| | Methyl ethyl ketone | No | Good |
| | Methyl n-propyl ketone | No | Good |
| | Methyl isobutyl ketone | No | Good |
| | 2-heptanone | No | Moderate |
| | 5-methyl-2-hexanone | No | Moderate |
| Carboxylic Acids | Formic acid | Yes | Poor |
| | Acetic acid | Yes | Good |
| | Propionic acid | Yes | Good |
| Aromatic Hydrocarbons | Toluene | No | * |
| | Xylene | No | * |
| Hydrocarbons | Hexane | No | Poor |
| | Cyclohexane | No | Poor |
| Chlorinated Aliphatics | Dichloromethane | No | Good |
| | Chloroform | No | Moderate |
| | 1,1,1-trichloroethane | No | Good |
| | 1,2-dichloroethane | No | Good |
| | 1,1,2-trichloroethane | No | Good |
| Aliphatic Nitrogen | Acetonitrile | Yes | Good |
| Halogenated Aromatics | Chlorobenzene | No | Moderate |

*Not effective at room temperature but suitable at elevated temperature.

Example 9

A sample of dry crude sorbic acid (dry basis weight 365 g, 93% sorbic acid) was divided into three portions. The first portion (100 pbw) was wetted with water (200 pbw) then slurried in 1,1,2-trichloroethane (200 pbw). Sorbic acid crystals were separated from the slurry mother liquor by filtration and the filter cake spray washed with a further aliquot of 1,1,2-trichloroethane (50 pbw).

The mother liquor and spent spray wash liquor obtained from solvent treatment of the first portion of wet crude were used to slurry a second portion of crude (dry basis weight 115 pbw). After separation of sorbic acid crystals from the second slurry by filtration, a second filter cake was obtained which was spray washed with another aliquot of 1,1,2-trichloroethane (50 pbw).

The mother liquor filtrate and spent spray wash liquor obtained from washing the second portion of crude sorbic acid were combined and used to slurry a third portion of sorbic acid (dry basis weight 150 pbw). Crystals of the third slurry were separated by filtration yielding a third filter cake which was spray washed with still another aliquot of fresh 1,1,2-trichloroethane (50 pbw).

After drying, the filter cakes obtained from washing each of the three portions of wet crude sorbic acid were weighed. The first filter cake weighed 82 pbw, the second 102 pbw, and the third 140 pbw, a total of 324 pbw. Thus, the total recovery of sorbic acid in this example was 95%.

Example 10

A portion of dry crude sorbic acid (dry basis assay 93.0% by weight) was divided into three portions which were subjected to solvent treatment according to the scheme described below.

The first portion (100 pbw dry basis) was wetted with water (50 pbw), then slurried in methyl isobutyl ketone (200 pbw), after which the crystalline sorbic acid was separated from the mother liquor by filtration. The filter cake was then washed by spraying it with additional methyl isobutyl ketone (50 pbw). In this instance, the spent spray wash liquor was maintained separate from the mother liquor which had constituted the slurry medium.

The second portion of dry crude sorbic acid (100 pbw) was slurried in the mother liquor filtrate obtained in the separation of sorbic acid crystals from the first slurry. The sorbic acid crystals of the second slurry were then recovered by filtration and spray washed with an additional aliquot of methyl isobutyl ketone (50 pbw), which was again kept separate from the second mother liquor.

The third portion of wet crude sorbic acid (dry basis weight 100 pbw) was slurried in the mother liquor obtained in the separation of sorbic acid from the second slurry. This mother liquor contained some solids. After separation of crystalline sorbic acid from the third slurry by filtration, the filter cake was spray washed with methyl isobutyl ketone (50 pbw).

Each filter cake was weighed. That obtained from solvent treatment of the first portion of crude sorbic acid was found to weigh 65.4 pbw, that obtained from treating the second portion of sorbic acid was found to weigh 86.0 pbw, and that obtained from treating the third portion was found to weigh 94.3 pbw, for a total weight of recovered sorbic acid of 245.0 pbw. This represented a recovery of 88%.

Example 11

A methyl n-propyl ketone mother liquor (27.7% solids) was obtained by slurrying samples of crude sorbic acid with methyl n-propyl ketone and separating the mother liquor from the sorbic acid crystals by filtration. A spent wash liquor was obtained by spray washing the filter cake with fresh methyl n-propyl ketone. The sorbic acid subjected to the solvent treatment operations had been produced by acid catalyzed depolymerization of a polyester produced by condensation of ketene and crotonaldehyde.

Another portion of dry crude sorbic acid (200 pbw; 93.0% assay), also produced by splitting a poly(3-hydroxy-4-hexenoate) polyester, was slurried in a slurry medium obtained by combining a portion of the mother liquor obtained from the previous solvent treatment operation (235 pbw), water (40 pbw), and a portion (65 pbw) of the spent wash liquor from the previous operation. Thereafter, the crystalline sorbic acid was separated by filtration, and the cake was washed with fresh methyl n-propyl ketone (65 pbw; saturated with water) and then with an additional quantity of water (80 pbw).

A further 200 pbw portion of the dry crude sorbic acid was slurried in a slurry medium comprised of the mother liquor (235 pbw) obtained from the preceding batch, water, and recycle spent wash liquor. Separation by filtration, washing with fresh methyl n-propyl ketone, and washing with fresh water were repeated in the above sequence.

Thereafter, two additional portions of crude sorbic acid were so processed. The total amount of sorbic acid obtained in the filter cakes of the last three iterations was determined to be 527.8 pbw. Thus, total overall recovery of sorbic acid for these three iterations was 94.6%. The mother liquor filtrate was analyzed by loss on drying, and found to contain 33.6% solids. These solids were analyzed and found to contain 34.9% sorbic acid.

Example 12

Dry crude sorbic acid (100 pbw, 93% sorbic acid) was wetted with water (20 pbw) and then slurried in 1,1,1-trichloroethane (300 pbw). The slurry was stirred for ten minutes and separated by centrifugation. The centrifuge cake was washed with fresh 1,1,1-trichloroethane (100 pbw) and the spent wash liquor maintained separate from the slurry medium mother liquor. Weight of the dry centrifuge cake was 84.3 pbw. After separation of an aqueous layer, the weight of the slurry medium mother liquor was 275.5 pbw and the weight of the spent wash liquor was 53.0 pbw.

The 275.5 pbw of mother liquor was mixed with a portion of the spent wash liquor sufficient to provide a recycle 1,1,1-trichloroethane slurry medium having a total weight of 300 pbw. A further portion of crude sorbic acid (100 pbw) was mixed with water (20 pbw) and the recycle slurry medium to provide a second slurry. This second slurry was again separated by centrifugation and the cake washed with fresh 1,1,1-trichloroethane (100 pbw). Mother liquor (271.0 pbw) and spent wash liquor (61.5 pbw) from the second batch were collected seaprately. The dry weight of the cake was 90.1 pbw.

Mother liquor from the second batch (271.0 pbw) was augmented with sufficient spent wash liquor from the second washing operation to provide a total of 300 pbw of recycle slurry medium for a third batch.

In the third batch, another 100 pbw portion of crude sorbic acid was solvent treated in a manner identical to that described above for the first two operations.

Using a portion (200.0 pbw) of the mother liquor from the third batch (augmented with sufficient spent wash liquor and fresh 1,1,1-trichloroethane to provide 300 pbw of slurry medium), a fourth solvent treatment operation was carried out on a fourth 100 pbw portion of crude sorbic acid.

The results of these sequential solvent treatment operations are set forth in Table V, 1,1,1-trichloroethane being indicated as "TCE". The solvent treated sorbic acid cakes from each of the successive

EP 0 153 292 B1

treatments were determined to be of equal quality by light transmittance measurements (made according to the procedure described hereinafter).

TABLE V

| (Measured in pbw) | Solvent treatment operation number (n) | | | |
|---|---|---|---|---|
| | 1 | 2 | 3 | 4 |
| Crude Sorbic Acid | 100 | 100 | 100 | 100 |
| Slurry Medium: | | | | |
| Water | 20 | 20 | 20 | 20 |
| Fresh TCE | 300 | — | — | 30 |
| Mother Liquor (Batch n-1 | — | 275.5 | 271.0 | 200 |
| Spent Wash Liquor (Batch n-1) | — | 24.5 | 29.0 | 70.0 |
| TCE Spray Wash | 100 | 100 | 100 | 100 |
| Mother Liquor Recovered (Batch n) | 275.5 | 271.0 | 259.0 | 270.0 |
| Wash Liquor Recovered (Batch n) | 53.0 | 61.5 | 70.0 | — |
| Washed Sorbic Acid Cake Weight | 84.3 | 90.1 | 91.3 | 90.8 |

Using the procedure described at the outset of this example, four portions of crude sorbic acid (100 pbw each, 93% sorbic acid) were solvent treatment in sequential solvent slurrying and centrifuge washing operations, using dichloromethane as the solvent. Mother liquor obtained from the first operation was mixed with spent cake wash liquor and used as a recycle slurry medium for the second batch. Thereafter the mother liquor from each operation was used in the slurry medium for the immediately subsequent operation. In each instance, the slurry medium was augmented by inclusion of spent wash liquor and fresh dichloromethane so that the total amount of slurry medium was 300 pbw. After the third batch, a portion (64 pbw) of the mother liquor was purged.

The amounts of materials used and results obtained in these solvent treatment operations are set forth in Table VI. No significant batch-to-batch variation in solvent-treated sorbic acid quality was observed.

TABLE VI

| (Measured in pbw) | Solvent treatment operation number (n) | | | |
|---|---|---|---|---|
| | 1 | 2 | 3 | 4 |
| Crude Sorbic Acid | 100 | 100 | 100 | 100 |
| Slurry Medium: | | | | |
| Water | 20 | 20 | 20 | 20 |
| Fresh Dichloromethane | 300 | 20 | 24 | 79 |
| Mother Liquor (Batch n-1) | — | 262 | 261 | 200 |
| Spent Wash Liquor (Batch n-1) | — | 18 | 15 | 21 |
| Dichloromethane Spray Wash | 100 | 100 | 100 | 100 |
| Mother Liquor Recovered (Batch n) | 262 | 261 | 264 | 264 |
| Washed Sorbic Acid Cake Weight | 77.2 | 90.5 | 91.7 | 88.7 |

12

Three additional portions of crude sorbic acid (150 pbw each) were separately slurried in water (850 pbw), the sorbic acid separated from the slurry by filtration, and each filter cake washed with 90% ethanol solution (150 pbw for each batch). Weights of washed sorbic acid obtained from these three operations were 118.2 pbw, 117.9 pbw, and 119.5 pbw, respectively. Solvent-treated sorbic acid purity did not vary significantly from batch to batch but was slightly lower than that of the washed cakes in Tables V and VI.

Example 13

A series of portions of wet sorbic acid crude were solvent treated for removal of impurities. In each instance, the crude sorbic acid was slurried in dichloromethane, the crystalline sorbic acid was separated from the slurry by centrifugation, and the centrifuge cake washed with fresh dichloromethane. Mother liquor filtrate and spent wash liquor were collected separately. In each operation subsequent to the first, the slurry medium comprised mother liquor filtrate from the preceding operation combined with a sufficient portion of the wash liquor from the preceding operation to provide the desired quantity of organic solvent medium. Set forth in Table VII are the proportions of components utilized in the preparation of slurry and washing of the filter cake for each solvent treatment operation.

TABLE VII

| | Solvent treatment operation number (n) | | | | | |
|---|---|---|---|---|---|---|
| (Measured in pbw) | 1 | 2 | 3 | 4 | 5 | 6 |
| Wet Crude Sorbic Acid | 240 | 240 | 240 | 240 | 240 | 240 |
| Slurry Medium Fresh Dichloro- methane | 400 | — | — | — | — | — |
| Mother Liquor (Batch n-1) | | | | 220[b] | 220[b] | 220[b] |
| Spent Spray Liquor (Batch n-1) | | 400[a] | 400[a] | 180 | 180 | 180 |
| Dichloromethane Spray Wash | 200 | 200 | | 200 | 200 | 200 |
| Water Spray Wash | 200 | 200 | | 200 | 200 | 200 |

[a] All mother liquor plus sufficient wash liquor to provide 400 pbw of slurry medium.
[b] Remainder of mother liquor effectively purged to mother liquor reservoir.

A portion of the combined washed filter cake of batches four, five, and six (239 pbw) was mixed with water (296 pbw) and 45% by weight potassium hydroxide solution (approximately 265 pbw). The resulting solution had a pH of approximately 10.3. A 200 pbw portion of this solution was treated with activated carbon (1.6. pbw) and filtered. A 100 pbw portion of the filtered solution was treated with a further portion of 0.8 pbw carbon and filtered. The doubly treated solution was tested for transparency by measuring its transmittance of 400 nanometer wavelength light, using a Bausch & Lomb Spectronic 20 spectrophotometer. The measured transmittance was 89% as compared to a deionized water standard.

Three additional batches of crude sorbic acid were solvent treated by slurrying in ethyl acetate, separation of the solids by centrifugation, and washing of the centrifuge cake with ethyl acetate and water. Recycle mother liquor was used as the slurry medium in the second batch and a mixture of recycle mother liquor and spent wash liquor, both from the second batch, was used as the slurry medium for the third batch. The amounts of crude sorbic acid, solvent, mother liquor, spent wash liquor, and water used in each of these batches are set forth in Table VIII.

TABLE VIII

| (Measured in pbw) | Operation batch number (n) | | |
|---|---|---|---|
| | 1 | 2 | 3 |
| Wet Crude Sorbic Acid | 240 | 240 | 240 |
| Slurry Medium (Ethyl Acetate) | | | |
|   Fresh Solvent | 300 | — | — |
|   Mother Liquor | | | |
|     (Batch n-1) | — | 225 | 225 |
|   Spent Wash | | | |
|     (Batch n-1) | — | 75 | 75 |
| Spray Wash (Ethyl Acetate) | | | |
|   Ethyl Acetate | 100 | 75 | 75 |
|   Water | 100 | 75 | 75 |

From the third batch in each of the series of operations recorded in Tables VII and VIII, a sample (125 pbw) of dry, washed sorbic acid powder was taken, and this sample was mixed with water (148 pbw) and 45% by weight potassium hydroxide (133 pbw). The resulting solution was adjusted to a pH of 10.5.

A portion (200 pbw) of each of these solutions was mixed with activated carbon (1.6 pbw) and the carbon slurry stirred for 15 minutes before the carbon was removed by filtration. The resulting filtrates were measured for transmittance of 400 nanometer wave length light.

A 100 pbw specimen of each these filtrates was then slurried with an additional portion of activated carbon (0.8 pbw) and the resulting slurry stirred for 15 minutes before the carbon was again separated by filtration. After carbon separation, light transmittance observations were again made on the filtrates. Set forth in Table IX are the results of the light transmittance tests.

TABLE IX

Light transmittance of sorbic acid/potassium solution*

| | Single carbon treatment | Double carbon treatment |
|---|---|---|
| Ethyl Acetate Washed | 83% | 88% |
| Dichloromethane Washed | 85% | 89% |

*400 nanometer wavelength light.

Example 14

Dry crude sorbic acid (200 pbw) was slurried with water (40 pbw) and ethyl acetate (300 pbw). Sorbic acid crystals were separated from the slurry by centrifugation, and the cake was washed with a 97:3 w/w ethyl acetate/water mixture (80 pbw) and then with water (80 pbw). The mother liquor obtained by separating the crude sorbic acid from the slurry was combined with a portion of spent ethyl acetate cake wash liquor and recycled for use in slurrying an additional 200 pbw portion of dry crude sorbic acid. This slurry was also separated by centrifugation and the cake washed in the above manner. Beginning with the third batch, the process streams were brought into a steady state balance, with the slurry medium for each batch comprising a combination of recycled mother liquor (225 pbw) and spent was liquor (75 pbw) from the previous batch. Mother liquor in excess of that used in the recycle slurry medium was effectively purged from the system after each batch by allowing the excess to accumulate in a large mother liquor reservoir. The cake from the third batch was dissolved in a potassium hydroxide solution in the manner described in Example 13, and the resulting solution of potassium sorbate subjected to two-stage carbon treatment in the manner described in Example 13. The filtrate obtained after the first carbon treatment was determined to have a transmittance of 80% for 400 nanometer light, and the filtrate from the second carbon treatment was observed to have a transmittance of 86%.

Example 15

Dry crude sorbic acid (200 pbw, 93% sorbic acid) was wetted with water (40 pbw) and slurried in methyl isobutyl ketone (300 pbw). Sorbic acid crystals were separated from the slurry by centrifugation and the centrifuge cake was washed with methyl isobutyl ketone (80 pbw) and water (80 pbw).

A second batch of plant crude sorbic acid (200 pbw) was wetted with water (40 pbw) and slurried in

14

mother liquor (300 pbw) obtained from the first batch. Again, the cake was washed with methyl isobutyl ketone (80 pbw) and water (80 pbw). An additional six batches (batches 3 through 8) of sorbic acid (200 pbw dry basis each batch) were subjected to solvent treatment by first slurrying the crude sorbic acid in a slurry medium comprising water (40 pbw), mother liquor obtained from the previous batch (225 pbw) and spent methyl isobutyl ketone wash liquor (75 pbw) from the previous batch. After each batch, mother liquor in excess of 225 pbw was purged from the system. The filter cake in each batch was washed with methyl isobutyl ketone (80 pbw; water saturated) and water (80 pbw).

Centrifuge cake from the first three batches was combined and determined to have a weight of 454.5 pbw, a yield of 81.5%. Cake from the fourth batch was determined to have a weight of 160.9 pbw, a yield of 86.5%. Filter cake from batches 5 through 8 was combined and determined to have a weight of 700 pbw, a sorbic acid recovery of 94%.

Example 16

By slurrying in dichloromethane, separating the solids from the centrifuge cake by centrifugation, and washing the centrifuge cake in fresh solvent, crude crystalline sorbic acid was solvent treated for removal of tarry impurities. Tar laden mother liquor obtained in this operation was subjected to distillation in order to recover dichloromethane solvent and produce a tarry residue which could be purged from the process. Three distillation runs were made using a wiped film evaporator to which portions of mother liquor were continuously fed. Tarry residue was collected. Distillation conditions were varied among the three runs.

In the first two runs, the mother liquor used had been obtained from a multiple recycle slurry and cake wash centrifugation operation.

In the third run, the mother liquor feed had been obtained from a single slurry and centrifugation batch.

Distillation conditions and results obtained in the distillation runs of this example are set forth in Table X.

TABLE X

| | Run number | | |
|---|---|---|---|
| | 1 | 2 | 3 |
| Pressure | Atmospheric | −115 mm Hg | −115 mm Hg |
| Temperature | 105°C | 105°C | 105°C |
| Feed Rate | 12.4 pbw/min | 20.0 pbw/min | 25.8 pbw/min |
| Mother Liquor Charge | 502.5 pbw | 410.6 pbw | 619.5 pbw |
| Dichloromethane Distillate | 418.6 pbw | 356.4 pbw | 558.3 pbw |
| Tarry Residue | 65.3 | 45.55 | 46.65 |
| Recovery—Weight | 483.9 pbw | 402.0 pbw | 604.9 pbw |
| Recovery—Percent | 96.3 | 97.9 | 97.7 |

Example 17

Utilizing the method generally described in Example 16, crude sorbic acid was washed for removal of tarry impurities. The tar laden mother liquor was subjected to distillation in order to remove dichloromethane solvent and produce a tar residue for discard. Distillation was carried out in a still pot to which the feed was delivered continuously and solvent removed continuously until tars in the pot had accumulated to a pre-determined level. Feeding of the still was then terminated and the tars drained. Several distillation runs were made. All runs were conducted at atmospheric pressure with the pot submerged in a 94°C oil bath.

In each run, the pot temperature was measured periodically and found to range typically from about 45°C, once a substantial pool had been formed by delivery of feed to the pot, to 75°C to 80°C near the completion of the run. The material balance was determined for each run. The results of the runs of this example are set forth in Table XI.

TABLE XI

| | Run number | | | |
|---|---|---|---|---|
| | 1 | 2 | 3 | 4 |
| Feed Rate (pbw/min) | 8.45 | 15.8 | 22.0 | 32.7 |
| Feed Time (min/sec) | 45'52"* | 24'50" | 16'45" | 11'40" |
| Feed (pbw) | 387.6 | 396.96 | 366.76 | 381.80 |
| Dichloromethane Distillate (pbw) | 332.7 | 336.52 | 315.08 | 328.83 |
| Residue (pbw) | 48.7 | 51.07 | 46.88 | 49.34 |
| Total Recovery: Weight (pbw) | 381.4 | 387.59 | 361.96 | 378.17 |
| Percentage | 98.4 | 98.9 | 98.7 | 99.0 |

*Pot held in bath for an additional six minutes, until pot temperature reaches 74.8°C.

In view of the above, it will be seen that the several objects of the invention are achieved and other advantageous results attained.

As various changes could be made in the above processes and methods without departing from the scope of the invention, it is intended that all matter contained in the above description or shown in the accompanying drawings shall be interpreted as illustrative and not in a limiting sense.

**Claims**

1. A process for the preparation of purified sorbic acid comprising the steps of reacting crotonaldehyde with ketene in the presence of a catalyst for the preparation of a polyester of 3-hydroxy-4-hexenoic acid, depolymerizing the polyester with an aqueous mineral acid to precipitate a crude crystalline sorbic acid from an aqueous phase of a depolymerization reaction mixture; and separating the sorbic acid precipitate from said aqueous phase; characterised by contacting the crude crystalline sorbic acid with an organic solvent that comprises (i) a water-immiscible solvent selected from low molecular weight dihaloalkanes, low molecular weight trihaloalkanes, lower alkyl esters of low molecular weight alkanoic acids, low molecular weight ketones, and low molecular weight ethers, or (ii) a water-miscible solvent selected from lower molecular weight alcohols, lower molecular weight carboxylic acids, γ-butyrolactone, acetone and acetonitrile, said organic solvent being used in an amount which is effective for dissolving impurities formed in the preparation of the crude sorbic acid but under conditions such that no more than a minor proportion of said sorbic acid is dissolved, thereby producing a substantially purified crystalline sorbic acid and an organic liquor comprising said solvent and containing a substantial portion of said impurities; and mechanically separating said liquor from said crystalline sorbic acid.

2. A process according to Claim 1 wherein said solvent is substantially immiscible with water; crude crystalline sorbic acid is mixed with said solvent after separation of said precipitate from said aqueous phase, thereby producing a slurry comprising crystalline sorbic acid and a mother liquor, said mother liquor comprising said solvent and containing impurities removed from said crude sorbic acid, said slurry is delivered to a solid/liquid separation means and said mother liquor is thereby separated to produce a cake comprising crystalline sorbic acid.

3. A process according to Claim 2 wherein at least a portion of the separated mother liquor is recycled and mixed with crude sorbic acid in producing additional quantities of said slurry.

4. A process according to either of Claims 2 and 3 wherein said solvent is selected from dichloromethane, 1,1,1-trichloroethane, ethyl acetate, methyl ethyl ketone, methyl n-propyl ketone, methyl isopropyl ketone, and methyl isobutyl ketone.

5. A process according to any of Claims 1 to 4 wherein said crystalline sorbic acid is subjected to at least a two stage treatment with said solvent.

6. A process according to any of Claims 1 to 5 wherein said substantially purified sorbic acid is contacted with a basic alkali or alkaline earth metal compound in an aqueous medium, thereby producing an aqueous solution of an alkali or alkaline earth metal sorbate.

7. A process according to any of Claims 1 to 6 wherein the crude crystalline sorbic acid is contacted with said solvent at a temperature up to about 30°C.

8. A process for the preparation of purified sorbic acid comprising the steps of reacting crotonaldehyde with ketene in the presence of a catalyst for the preparation of a polyester of 3-hydroxy-4-hexenoic acid,

16

EP 0 153 292 B1

depolymerizing the polyester with an aqueous mineral acid to precipitate a crude crystalline sorbic acid from an aqueous phase of a depolymerization reaction mixture; characterised by mixing an organic solvent that comprises a water-immiscible solvent selected from the low molecular weight dihaloalkanes, low molecular weight trihaloalkanes, lower alkyl esters of low molecular weight alkanoic acids, low molecular weight ketones, and low molecular weight ethers, with said aqueous phase to contact said crude sorbic acid with said solvent which is effective for dissolving impurities formed in the preparation of the crude sorbic acid and which will dissolve no more than a minor proportion of said sorbic acid thereby producing a slurry containing sorbic acid, said aqueous phase, and a mother liquor, said mother liquor comprising said solvent and containing impurities removed from said crude sorbic acid, and separating a substantially purified crystalline sorbic acid.

9. A process according to Claim 8 wherein said organic solvent is substantially immiscible with water and said slurry is delivered to a solid/liquid separation means thereby producing a cake comprising crystalline sorbic acid, and a two-phase filtrate comprising said aqueous phase and said mother liquor.

10. A process according to Claim 9 wherein at least a portion of the separated mother liquor is recycled and mixed with crude sorbic acid in producing additional quantities of said slurry.

11. A process according to any of Claims 8 to 10 wherein said crystalline sorbic acid is subjected to at least a two stage treatment with said solvent.

12. A process according to any of Claims 8 to 11 wherein said solvent is selected from dichloromethane, 1,1,1-trichloroethane, ethyl acetate, methyl ethyl ketone, methyl n-propyl ketone, methyl isopropyl ketone, and methyl isobutyl ketone.

13. A process according to any of Claims 8 to 12 wherein said substantially purified sorbic acid is contacted with a basic alkali or alkaline earth metal compound in an aqueous medium, thereby producing an aqueous solution of an alkali or alkaline earth metal sorbate.

14. A process according to any of Claims 8 to 13 wherein the crude crystalline sorbic acid is contacted with said solvent at a temperature up to about 30°C.


**Patentansprüche**

1. Verfahren zur Herstellung von gereinigter Sorbinsäure durch Umsetzen von Crotonaldehyd mit Keten in Gegenwart eines Katalysators zur Herstellung eines 3-Hydroxy-4-hexensäurepolyesters, Entpolymerisieren des Polyesters mit einer wäßrigen Mineralsäure unter Ausfällung einer rohen kristallinen Sorbinsäure aus der wäßrigen Phase der Entpolymerisationsreaktionsmischung und Abtrennen des Sorbinsäureniederschlags von der wäßrigen Phase, dadurch gekennzeichnet, daß man die rohe kristalline Sorbinsäure mit einem organischen Lösungsmittel, das

(i) ein mit Wasser nicht mischbares Lösungsmittel, gewählt aus der Gruppe der Dihalogenalkane mit niedrigem Molekulargewicht, Trihalogenalkane mit niedrigem Molekulargewicht, Niedrigalkylester von Alkansäuren mit niedrigem Molekulargewicht, Ketone mit niedrigem Molekulargewicht und Ether mit niedrigem Molekulargewicht oder

(ii) ein mit Wasser mischbares Lösungsmittel, gewählt aus Alkoholen mit niedrigem Molekulargewicht, Carbonsäuren mit niedrigem Molekulargewicht, γ-Butyrolacton, Aceton und Acetonitril umfaßt, in Kontakt bringt, wobei das organische Lösungsmittel in einer Menge eingesetzt wird, die zur Lösung der während der Herstellung der rohen Sorbinsäure gebildeten Verunreinigungen wirksam ist, jedoch unter solchen Bedingungen, daß nicht mehr als ein geringer Teil der Sorbinsäure in Lösung geht, so daß eine im wesentlichen gereinigte kristalline Sorbinsäure und eine das Lösungsmittel umfassende und den wesentlichen Teil der Verunreinigungen enthaltende organische Flüssigkeit erhalten wird, und die Flüssigkeit von der kristallinen Sorbinsäure mechanisch abtrennt.

2. Verfahren nach Anspruch 1, dadurch gekennzeichnet, daß das Lösungsmittel im wesentlichen mit Wasser nicht mischbar ist und man die rohe kristalline Sorbinsäure mit dem Lösungsmittel nach Abtrennung des Niederschlags von der wäßrigen Phase vermischt, so daß man eine die kristalline Sorbinsäure und die Mitterlauge enthaltende Aufschlämmung erhält, wobei die Mitterlauge das Lösungsmittel umfaßt und de von der rohen Sorbinsäure entfernten Verunreinigungen enthält, die Aufschlämmung zu einer Feststoff-/Flüssigkeitstrennvorrichtung befördert und die Mutterlauge unter Bildung eines die kristalline Sorbinsäure umfassenden Kuchens abtrennt.

3. Verfahren nach Anspruch 2, dadurch gekennzeichnet, daß man mindestens einen Teil der abgetrennten Mitterlauge zurückführt und mit der rohen Sorbinsäure zur Bildung zusätzlicher Mengen an Aufschlämmung vermischt.

4. Verfahren nach einem der Ansprüche 2 und 3, dadurch gekennzeichnet, daß das Lösungsmittel aus Dichlormethan, 1,1,1-Trichlorethan, Ethylacetat, Methylethylketon, Methyl-n-propylketon, Methylisopropylketon und Methylisobutylketon gewählt ist.

5. Verfahren nach einem der Ansprüche 1 bis 4, dadurch gekennzeichnet, daß man die kristalline Sorbinsäure mindestens einer Zweistufenbehandlung mit dem Lösungsmittel unterwirft.

6. Verfahren nach einem der Ansprüche 1 bis 5, dadurch gekennzeichnet, daß man die im wesentlichen gereinigte Sorbinsäure mit einer basischen Alkali- oder Erdalkalimetallverbindung in einem wäßrigen Medium unter Bildung einer wäßrigen Lösung aus einem Alkali- oder Erdalkalimetallsorbat in Kontakt bringt.

17

7. Verfahren nach einem der Ansprüche 1 bis 6, dadurch gekennzeichnet, daß man die rohe kristalline Sorbinsäure mit dem Lösungsmittel bei einer Temperatur bis zu etwa 30°C in Kontakt bringt.

8. Verfahren zur Herstellung von gereinigter Sorbinsäure durch Umsetzen von Crotonaldehyd mit Keten in Gegenwart eines Katalysators zur Herstellung eines 3-Hydroxy-4-hexensäurepolyesters, Entpolymerisieren des Polyesters mit einer wäßrigen Mineralsäure unter Ausfallung einer rohen kristallinen Sorbinsäure aus der wäßrigen Phase der Entpolymerisationsreaktionsmischung, dadurch gekennzeichnet, daß man ein organisches Lösungsmittel, das ein mit Wasser nicht mischbares Lösungsmittel, gewählt aus der Gruppe der Dihalogenalkane mit niedrigem Molekulargewicht, Trihalogenalkane mit niedrigem Molekulargewicht, Niedrigalkylester von Alkansäuren mit niedrigem Molekulargewicht, Ketone mit niedrigem Molekulargewicht und Ether mit niedrigem Molekulargewicht umfaßt, mit der wäßrigen Phase mischt und die rohe Sorbinsäure mit dem Lösungsmittel, das zur Lösung der während der Herstellung der rohen Sorhinsäure gebildeten Verunreinigungen wirksam ist und nicht mehr als einen geringen Teil der Sorbinsäure löst, unter Bildung einer die Sorbinsäure, die wäßrige Phase und die Mutterlauge enthaltenden Aufschlämmung in Kontakt bringt, wobei die Mutterlauge das Lösungsmittel umfaßt und die von der rohen Sorbinsäure entfernten Verunreinigungen enthält, und die im wesentlichen gereinigte Sorbinsäure abtrennt.

9. Verfahren nach Anspruch 8, dadurch gekennzeichnet, daß das organische Lösungsmittel im wesentlichen mit Wasser nicht mischbar ist und man die Aufschlämmung zu einer Feststoff-/Flüssigkeitstrennvorrichtung unter Bildung eines die kristalline Sorbinsäure umfassenden Kuchens befördert, wobei das Zweiphasenfiltrat die wäßrige Phase und die Mutterlauge enthält.

10. Verfahren nach Anspruch 9, dadurch gekennzeichnet, daß man mindestens einen Teil der abgetrennten Mutterlauge zurückführt und mit der rohen Sorbinsäure zur Bildung zusätzlicher Mengen an Aufschlämmung vermischt.

11. Verfahren nach einem der Ansprüche 8 bis 10, dadurch gekennzeichnet, daß man die kristalline Sorbinsäure mindestens einer Zweistufenbehandlung mit dem Lösungsmittel unterwirft.

12. Verfahren nach einem der Ansprüche 8 bis 11, dadurch gekennzeichnet, daß das Lösungsmittel aus Dichlormethan, 1,1,1-Trichlorethan, Ethylacetat, Methylethylketon, Methyl-n-propylketon, Methylisopropylketon und Methylisobutylketon gewählt ist.

13. Verfahren nach einem der Ansprüche 8 bis 12, dadurch gekennzeichnet, daß man die im wesentlichen gereinigte Sorbinsäure mit einer basischen Alkali- oder Erdalkalimetallverbindung in einem wäßrigen Medium unter Bildung einer wäßrigen Lösung aus einem Alkali- oder Erdalkalimetallsorbat in Kontakt bringt.

14. Verfahren nach einem der Ansprüche 8 bis 13, dadurch gekennzeichnet, daß man die rohe kristalline Sorbinsäure mit dem Lösungsmittel bei einer Temperatur bis zu etwa 30°C in Kontakt bringt.

## Revendications

1. Procédé de préparation d'acide sorbique purifié comprenant les stades de faire réagir le crotonaldéhyde avec le cétène en présence d'un catalyseur pour la préparation d'un polyester d'acide 3-hydroxy-4-hexénoïque, de dépolymériser le polyester au moyen d'un acide minéral aqueux pour faire précipiter un acide sorbique cristallin brut à partir d'une phase aqueuse d'un mélange de réaction de dépolymérisation et de séparer le précipité d'acide sorbique de la phase aqueuse, caractérisé par la mise en contact de l'acide sorbique cristallin brut avec un solvant organique qui comprend (i) un solvant non miscible à l'eau choisi parmi les dihaloalcanes de bas poids moléculaire, les trihaloalcanes de bas poids moléculaire, les esters alcoyliques inférieurs d'acides alcanoïques de bas poids moléculaire, les cétones de bas poids moléculaire et les éthers de bas poids moléculaire, ou (ii) un solvant miscible à l'eau choisi parmi les alcools de poids moléculaire inférieur, les acides carboxyliques de poids moléculaire inférieur, la γ-butyrolactone, l'acétone et l'acétonitrile, ce solvant organique étant utilisé en une quantité qui est efficace pour dissoudre les impuretés formées lors de la préparation de l'acide sorbique brut, mais dans des conditions telles qu'il ne se dissolve pas plus d'une proportion mineure de l'acide sorbique, de manière à donner un acide sorbique cristallin sensiblement purifié et une liqueur organique comprenant le solvant et contenant une fraction sensible des impuretés; et la séparation mécanique entre la liqueur et l'acide sorbique cristallin.

2. Procédé suivant la revendication 1, dans lequel le solvant est sensiblement non miscible à l'eau; l'acide sorbique cristallin brut est mélangé avec le solvant après que le précipité a été séparé de la phase aqueuse, de manière à donner une dispersion comprenant de l'acide sorbique cristallin et une liqueur mère, la liqueur mère comprenant le solvant et contenant les impuretés éliminées de l'acide sorbique brut; la dispersion est amenée à un dispositif de séparation solide/liquide et la liqueur mère est ainsi séparée pour donner un gâteau comprenant de l'acide sorbique cristallin.

3. Procédé suivant la revendication 2, dans lequel au moins une fraction de la liqueur mère séparée est recyclée et mélangée avec de l'acide sorbique brut pour la production de nouvelles quantités de la dispersion.

4. Procédé suivant la revendication 2 ou 3, dans lequel le solvant est choisi parmi le dichlorométhane, le 1,1,1-trichloroéthane, l'acétate d'éthyle, la méthyléthylcétone, la méthyl-n-propylcétone, la méthylisopropylcétone, et la méthylisobutylcétone.

5. Procédé suivant l'une quelconque des revendications 1 à 4, dans lequel l'acide sorbique cristallin est soumis à un traitement en au moins deux stades du moyen du solvant.

6. Procédé suivant l'une quelconque des revendications 1 à 5, dans lequel l'acide sorbique sensiblement purifié est mis en contact avec un composé basique de métal alcalin ou alcalino-terreux dans un milieu aqueux, de manière à donner une solution aqueuse d'un sorbate de métal alcalin ou alcalino-terreux.

7. Procédé suivant l'une quelconque des revendications 1 à 6, dans lequel l'acide sorbique cristallin brut est mis en contact avec le solvant à une température s'élevant jusqu'à environ 30°C.

8. Procédé de préparation d'acide sorbique purifié comprenant les stades de faire réagir le crotonaldéhyde avec le cétène en présence d'un catalyseur pour la prépara;tion d'un polyester d'acide 3-hydroxy-4-hexénoïque, de dépolymériser le polyester au moyen d'un acide minéral aqueux pour faire précipiter un acide sorbique cristallin brut à partir d'une phase aqueuse d'un mélange de réaction de dépolymérisation, caractérisé par le mélange d'un solvant organique qui comprend un solvant non miscible à l'eau choisi parmi les dihaloalcanes de bas poids moléculaire, des trihaloalcanes de bas poids moléculaire, les esters alcoyliques inférieurs d'acides alcanoïques de bas poids moléculaire, les cétones de bas poids moléculaire et les éthers de bas poids moléculaire avec la phase aqueuse pour mettre l'acide sorbique brut en contact avec le solvant qui est efficace pour dissoudre les impuretés formées lors de la préparation de l'acide sorbique brut et qui ne dissoudra pas plus d'une proportion mineure de l'acide sorbique, de manière à donner une dispersion contenant de l'acide sorbique, la phase aqueuse et une liqueur mère, la liqueur mère comprenant le solvant et contenant les impuretés éliminées de l'acide sorbique brut; et la séparation d'un acide sorbique cristallin sensiblement purifié.

9. Procédé suivant la revendication 8, dans lequel le solvant organique est sensiblement non miscible à l'eau et la dispersion est amenée à un dispositif de séparation solide/liquide de manière à donner un gâteau comprenant de l'acide sorbique cristallin, et un filtrat biphasique comprenant la phase aqueuse et la liqueur mère.

10. Procédé suivant la revendication 9, dans lequel au moins une fraction de la liqueur mère séparée est recyclée et mélangée avec de l'acide sorbique brut pour la production de nouvelles quantités de la dispersion.

11. Procédé suivant l'une quelconque des revendications 8 à 10, dans lequel l'acide sorbique cristallin est soumis à un traitement en au moins deux stades au moyen du solvant.

12. Procédé suivant l'une quelconque des revendications 8 à 11, dans lequel le solvant est choisi parmi le dichlorométhane, le 1,1,1-trichloroéthane, l'acétate d'éthyle, la méthyléthylcétone, la méthyl-n-propylcétone, la méthylisopropylcétone et la méthylisobutylcétone.

13. Procédé suivant l'une quelconque des revendications 8 à 12, dans lequel l'acide sorbique sensiblement purifié est mis en contact avec un composé basique de métal alcalin ou alcalino-terreux dans un milieu aqueux, de manière à donner une solution aqueuse d'un sorbate de métal alcalin ou alcalino-terreux.

14. Procédé suivant l'une quelconque des revendications 8 à 13, dans lequel l'acide sorbique cristallin brut est mis en contact avec le solvant à une température s'élevant jusqu'à environ 30°C.

# FIG. 1

SOLVENT MAKE-UP

CRUDE SORBIC → RESLURRY TANK

SOLVENT STORAGE

H₂O TO STILL

MOTHER LIQUOR STILLPOT

WATER WASH → TAR REMOVAL CENTRI- FUGE

WASHED CAKE

TAR/BOTTOMS PURGE

SPENT WASH LIQUOR

SPENT WATER WASH

MOTHER LIQUOR

WASH SEPARATOR

MOTHER LIQUOR SEPARATOR

H₂O LAYER

ORG. LAYER

H₂O LAYER

ORG. LAYER

WASH TANK

MOTHER LIQUOR TANK

SOLVENT STRIPPER

# FIG. 2

# FIG. 3

POLYESTER

POLYESTER
DEPOLYMER-
IZATION

HCl
RECYCLE

HCl

SOLVENT
STORAGE

WASHED
CRUDE

SEPARATOR

MOTHER
LIQUOR
STORAGE

MOTHER LIQUOR STILLPOT

PURGE

TAR
PURGE